# EUROPÄISCHE PATENTANMELDUNG

(11) **EP 0 647 441 A1**
(43) Veröffentlichungstag der Anmeldung: **12.04.1995**
(21) Anmeldenummer: 93103754.3
(22) Anmeldetag: 09.03.1993
(51) Int. Cl.: A61F 5/56

(54) **Halsband mit elektronischer Baueinheit, die durch Akustik das Schnarchen verhindert**

(71) Anmelder: Kowoll, Dieter, D-81249 München (DE)
(72) Erfinder: Kowoll, Dieter, W-8000 München 60 (DE); Kowoll, Hubert, W-8960 Kempten/Allgäu (DE)

(57) **Zusammenfassung**

Vor dem Schlafengehen wird die Halsbandplatte ( 1 ) bequem, mit der viereckigen Öffnung ( 13 ) zum Kehlkopf, an den den Hals gelegt. Das weiche Band ( 7 ) wird um den Nacken gelegt und das Ende mit dem Klettverschluß ( 10 ) verbunden. Das andere Ende ( 11 ) haftet nach wie vor an dem Klettverschluß, das als Spiegelbild von der gegenüberliegenden Seite ( 10 ) ebenfalls mit drei Stück Hohlnieten ( 9 ) an der Halsbandplatte befestigt ist. Vor dem Einschlafen wird die Elektronik mittels Schalter ( 3 ) in Betrieb gesetzt. Beim Einschalten ist ein kurzer Piepton hörbar, der die Betriebsbereitschaft anzeigt. Wenn nun das Mikrofon ( 4 ) einen Schnarchton empfängt, geht ein Impuls zur Elektronik ( 6 ) und diese löst einen kurzen - etwa 0,3 bis 0,4 Sekunden langen - Piepton im Summer ( 5 ) aus. Der Schnarcher nimmt den Ton durch zwei 3 mm große Öffnungen an der Innenseite des Halsbandes im Unterbewußtsein wahr, und somit wird das Schnarchen im Ansatz abgebrochen. Das gleiche gilt bei etwaigem Sprechen. Eine Knopfbatterie 3 Volt ( 2 ) versorgt die Anlage über Kabel, die im Kabelschacht ( 8 ) untergebracht sind, mit Strom. Im Bedarfsfalle kann die Batterie durch eine dafür vorgesehene Klappe an der Innenseite der Halsbandplatte leicht ausgewechselt werden.

## Beschreibung

Die Erfindung betrifft eine Halsbandplatte, in der eine Knopfbatterie 3 Volt, ein Schiebeschalter, ein Mikrofon, ein Summer und eine elektronische Baueinheit integriert ist. Diese Halsbandplatte, aus hochelastischem Kunststoff ( Kautschuklegierung ) , wird vor dem Schlafengehen mit dem Klettverschluß locker um den Hals gelegt. Beim Einschalten der Anlage wird ein kurzer Piepton ausgelöst, der die Betriebsbereitschaft anzeigt. In der ersten Funktion wird sofort ein kurzer Piepton ausgelöst, wenn ein Schnarchgeräusch ertönt. Das Schnarchen wird somit sofort im Ansatz abgebrochen. In der zweiten Funktion wird etwaiges Sprechen auf die gleiche Weise unterbrochen. Während des Schlafens ist die Körperlage hierbei ohne Bedeutung.

Das Schnarchen ist ein ernstes Thema , auch wenn unzählige Witze darüber erzählt werden. Mancher Scheidungsanwalt kann aber berichten, wie viele Ehen unter anderem deshalb gefährdet sind , weil einer der Partner ein starker Schnarcher ist. Diesen Hinweis brachte der Bayerische Rundfunk im "Musikjournal" unter dem Titel : " Antischnarchgymnastik " von Ingo Traub, am 11.12.1979. Weiterhin wurden einige Übungen angeboten, um Mund- und Rachenmuskulatur zu kräftigen. Die Ernsthaftigkeit dieses Themas wird immer wieder betont, wie auch im Familienfunk ( BR 1 ) am 02.10.1991 um 11.30 Uhr , mit dem Hinweis auf die Broschüre von Dr. Lippmann, in der das Schnarchen behandelt wird.

In der Broschüre: "Das Schnarchen" von Peter Weidemann , Zwillings-Verlags GmbH, 2080 Pinneberg, werden Medikamente ( Tropfen , Pulver und Kapseln ) gegen Schnarchen über einen Warenvertrieb in Berlin angeboten.

Auch das Fernsehen hat sich wiederholt, unter anderem am 12.11.1991 mit dem Schnarchen befaßt. In der Sendereihe " Die Sprechstunde " unter der Leitung von Frau Dr. Antje- Katrin Kühnemann, mit dem Titel: " Gefährliche Nächte - Schnarchen " im BR 3 um 20.15 Uhr wurde das Schnarchen behandelt. Ausgehend von der Vermutung , daß die Hälfte der Bevölkerung schlecht schläft und davon ein großer Teil mit dem Schnarchen zusammenhängt, sind in Deutschland etwa dreihunderttausend Menschen von dieser Schlafstörung befallen. Die Auswirkungen gehen vom Leistungsabfall am Tage, über den Familienfrieden bis hin zur Ehetrennung. Vorher jedoch versucht man getrennt zu schlafen, denn das Schnarchen kann eine Geräuschkulisse von bis zu 90 Dezibel erzeugen, die mit einem vorbeifahrenden Motorrad vergleichbar ist. Der Oberarzt Dr. med. Karl Handschuh vom HNO- Klinikum rechts der Isar in München, schließt bei einem klinischen Befund eine Operation nicht aus. Es sei ein erheblicher Eingriff und die Operation bietet keine Garantie auf Erfolg. Auch Medikamente wie Beruhigungs- und Schlafmittel haben eher eine schnarchverstärkende Wirkung.

In dieser Sendung wurde letztlich als technischer Fortschritt ein Apparat mit der Bezeichnung " Sleep Easy III " vorgestellt. Über eine Atemmaske wird dabei von einem Kompressor , der von einem Elektromotor angetrieben wird, ein Luftstrom erzeugt. Diese Überdruckbeatmung hält die Atmungsorgane offen. Es wurde jedoch darauf hingewiesen, daß dabei Entzündungen der Nasenwände auftreten können.

Es ist offensichtlich, daß die Medizin bemüht ist , das Schnarchen als Schlafstörung bis zur bedrohlichen Erkrankung, verstärkt zu behandeln. Allerdings wird bei den drei Möglichkeiten, operativ, medikamentös und mit medizinisch- technischen Apparaten , auf nicht unerhebliche Nebenwirkungen hingewiesen. Die Videoaufzeichnung dieser TV- Sendung liegt vor.

Die lokale Presse in Kempten: " Allgäuer Zeitung " vom 21.02.1992 berichtete unter dem Titel : " Im Labor Schlafstörung auf der Spur " u.a. von einer Nasenmaske, die durch Überdruckbeatmung die verengten Atemwege offenhalten soll. Operative Eingriffe oder Medikamente seien weitere Behandlungsmöglichkeiten. Auch das Wochenblatt: " extra " vom 09.07.1992 schreibt in diesem Zusammenhang: " Wer schläft und schnarcht, lebt unter Umständen gefährlich " , sagt die AOK. Hier wird auch das Beatmungsgerät erwähnt. Das Deutsche Fernsehen hat sich meines Erachtens in der letzten Zeit verstärkt mit dem Thema Schnarchen befaßt. Der Sender SAT 1 hat in der Sendung: " Schreinemakers live " am 04.11.1992 um 22.25 Uhr u.a. über die Sauerstoffmaske berichtet; Bei dieser Anlage ist der Motor so laut, daß der Partner seperat schlafen muß. Auch am 30.12.1992 hat Frau Schreinemakers in Ihrer Live- Sendung unter anderem das Thema Schnarchen behandelt. Dabei hat Sie das Ehepaar Siebert vorgestellt. Nachdem Herr Siebert 20 Jahre lang schnarchte und alle Mühen ohne Erfolg waren, hatte er sich nun das Gaumenzäpfchen (Gaumensegel ) operativ entfernen lassen . Am 05.02.1993 brachte der Sender " Südwest 3 " um 19.00 Uhr in der Sendung " Hallo , wie geht's " mit der Moderatorin Frau Marianne Hoehl, ein Interview mit dem Internisten Dr. Heinrich Becker, der den Patienten H. Merkl vorgestellt hat. Diesem Patienten hat nur noch die Atemmaske geholfen.

Um dieses Problem human zu lösen, ist das " Anti- Schnarch- Halsband " entwickelt worden. Die Erfindung ist im Sektor " Akustik " angesiedelt, da das Schnarchen auf dem gleichen Gebiet liegt. Der Vorteil hierbei liegt darin, daß weder ein operativer Eingriff noch medikamentöse Therapie nötig ist. Es trägt auch nicht gerade zu einer beruhigenden Atmosphäre im Schlafzimmer bei, wenn ein medizinisch- technisches Elektrogerät aufgestellt und an den Mund angeschlossen wird und fast den Eindruck einer Intensivstation vermittelt. Auch muß der Schnarcher bei jedem Schnarchton keinen Elektroschock erleiden.

Im folgenden wird die Erfindung anhand von lediglich einer den Ausführungsweg darstellenden Zeichnung näher erläutert.

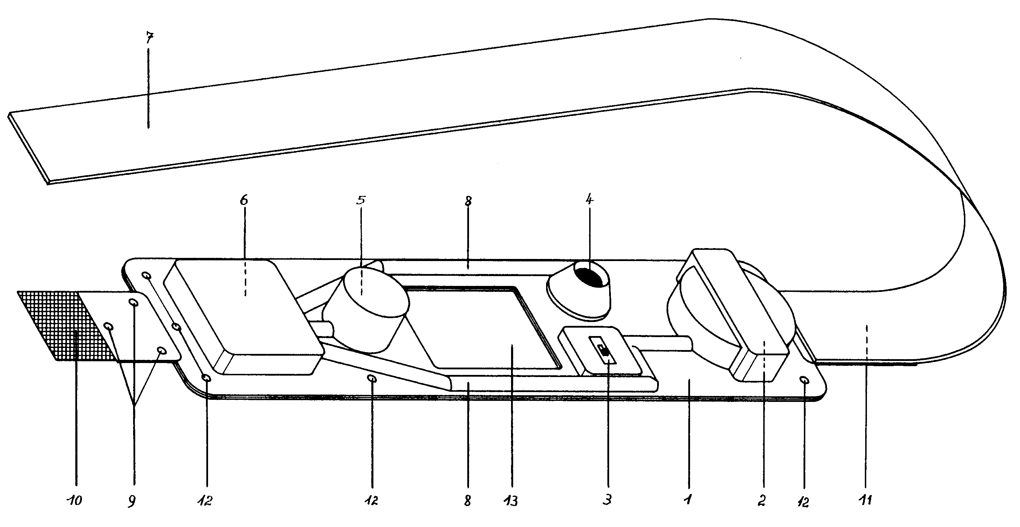

Die Zeichnung zeigt in perspektivischer Darstellung das erfindungsgemäße " Anti- Schnarch- Halsband " im Maßstab 1:1 mit integrierten Bauteilen. Die Halsbandplatte ( 1 ) ist auf der Rückseite zum Teil verschlossen. Eine 3-Volt Knopfbatterie ( 2 ) ist als Stromversorgung mit einer Kunststoffhalterung untergebracht. Der Strom wird mittels einer Litze im Kabelschacht ( 8 ) über einen Schiebeschalter ( 3 ) zur elektronischen Baueinheit ( 6 ) geleitet. Das Mikrofon ( 4 ) leitet bei einem Schnarchton die Impulse ebenfalls über eine Litze im Kabelschacht ( 8 ) zur elektronischen Baueinheit (6). Von hier wird der Summer ( 5 ), auch über eine Litze im Kabelschacht, angesteuert. An der Lasche der Halsbandplatte (1) -linksist das gerippte Teil des Klettverschlusses ( 10 ) mit drei Hohlnieten ( 9 ) fest vernietet. Das gleiche gilt als Spiegelbild auch für die rechte Seite ( 11 ), ist jedoch bereits mit dem Klettverschluß verbunden. Das weiche Klettverschlußband ( 7 ) gilt als Halterung des Halsbandes und wird um den Nacken gelegt. Das Ende vom Band ( 7 ) kann, dem Halsumfang entsprechend , mit dem Klettverschluß ( 10 ) verbunden werden.

## Patentansprüche

1. Halsband mit elektronischer Baueinheit, die durch Akustik das Schnarchen verhindert, wobei die Halsbandplatte ( 1 ) aus hochelastischem Kunststoff ist. Die Rückseite ist zum Teil geschlossen und hat Führungsstifte ( 12 ), die in die Bohrungen der Vorderseite eingeführt sind.

2. Halsband gemäß Anspruch 1 , dadurch gekennzeichnet, daß eine Knopfbatterie 3 Volt ( 2 ) mittels Litze im Kabelschacht ( 8 ) über einen Flachschiebeschalter ( 3 ) den Betriebsstrom zur Platine ( 6 ), die mit Bauteilen bestückt ist, liefert.

3. Halsband gemäß Anspruch 2, dadurch gekennzeichnet, daß das Mini- Mikrofon ( 4 ) bei Anregung die Impulse mittels Litze im Kabelschacht ( 8 ) zur elektronischen Baueinheit ( 6 ) leitet. Dabei wird, mittels Litze im Kabelschacht ( 8 ), der Summer (5) angeregt und gibt jeweils einen Piepton.
